(19) Europäisches Patentamt
European Patent Office
Office européen des brevets

(11) **EP 1 484 298 A1**

(12) **EUROPEAN PATENT APPLICATION**
published in accordance with Art. 158(3) EPC

(43) Date of publication:
**08.12.2004 Bulletin 2004/50**

(51) Int Cl.[7]: **C07B 33/00**, C07C 407/00,
C07C 409/10, C07C 409/12

(21) Application number: **03703246.3**

(22) Date of filing: **07.02.2003**

(86) International application number:
**PCT/JP2003/001286**

(87) International publication number:
**WO 2003/068711 (21.08.2003 Gazette 2003/34)**

(84) Designated Contracting States:
**AT BE BG CH CY CZ DE DK EE ES FI FR GB GR
HU IE IT LI LU MC NL PT SE SI SK TR**
Designated Extension States:
**AL LT LV MK RO**

(30) Priority: **15.02.2002 JP 2002038199**

(71) Applicant: **Sumitomo Chemical Company,
Limited
Osaka-shi Osaka 541-8550 (JP)**

(72) Inventors:
• **SEO, Tateo
Chuo-ku, Chiba-shi, Chiba 260-0042 (JP)**
• **OKU, Noriaki
Ichihara-shi, Chiba 290-0035 (JP)**

(74) Representative: **Duckett, Anthony Joseph
J.A. Kemp & Co.,
14 South Square,
Gray's Inn
London WC1R 5JJ (GB)**

(54) **PROCESS FOR PERFORMING OXIDATION REACTION**

(57) A process for oxidizing a liquid component with an oxygen-containing gas, wherein the oxidation is carried out under a condition of an agitation power per unit volume of 10.0 to 500 kg·m/sec/m³.

Fig.1

## Description

### TECHNICAL FIELD

The present invention relates to a process for performing an oxidation reaction. More particularly, the present invention relates to a process for oxidizing a liquid component with an oxygen-containing gas, wherein the oxidation process has an excellent characteristic that high efficiency of the oxidation reaction can be realized.

### BACKGROUND ART

Liquid phase oxidation processes of organic compounds using molecular oxygen in air or another mixed gas, are important chemical reactions in chemical industries, and in these oxidation reactions, oxygen reacts a reactant in a liquid phase while oxygen in a gas is dissolving in the liquid phase. However, in an industrial operation, the transfer of oxygen from the gas phase to the liquid phase is an important section and a common problem relating to mixing in liquid oxidation reactions.

As representative examples of the liquid phase oxidation in the industries, liquid phase auto-oxidations for converting hydrocarbons into organic peroxides by oxidation are particularly important, and specially, a conversion into t-butyl hydroperoxide by oxidation of isobutene, a conversion into cumene hydroperoxide by oxidation of cumene, a conversion into meta-diisopropyl benzene hydroperoxide by oxidation of meta-diisopropyl benzene, a conversion into ethyl benzene hydroperoxide by oxidation of ethyl benzene, a conversion into cymene hydroperoxide by oxidation of cymene and the like are important reactions.

Further, the liquid phase auto-oxidation is also adopted in a method in which anthraquinone is used as a reaction medium, which is industrially conducted at present as a major production process of hydrogen peroxide.

### DISCLOSURE OF THE INVENTION

Under such situations, the subject to be solved by the present invention is to provide a method for oxidizing a liquid component with an oxygen-containing gas, and a method for performing the oxidation reaction having an excellent characteristic that high efficiency of the oxidation can be realized.

Namely, the present invention relates to a method for oxidizing a liquid component with an oxygen-containing gas, wherein the oxidation is carried out under a condition of an agitation power per unit volume of 10.0 to 500 kg·m/sec/m$^3$.

### BRIEF DESCRIPTION OF THE DRAWING

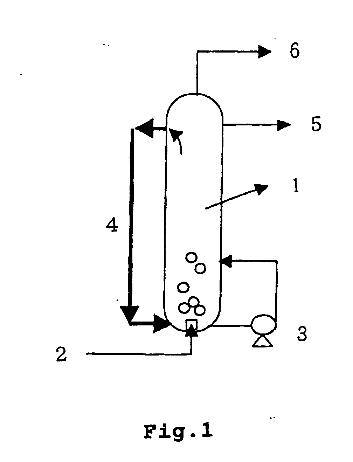

Fig.1 is a figure showing an outline of a reactor used in Example 1.

1. Bubble column, 2. Material feeding line, 3. Pump for outside circulating, 4.Liquid outside-circulating line, 5.Line for drawing reaction liquid, 6. Unreacted gas

### BEST MODE FOR CARRYING OUT THE INVENTION

The present invention can preferably apply to the oxidation of the liquid component with the oxygen-containing gas as described above. Further, in the production of an organic peroxide according to auto-oxidation of the above-described hydrocarbon, emulsion-oxidation is carried out for improving the yield of hydroperoxide to be produced. The emulsion-oxidation means oxidation carried out under a condition in which the liquid component is emulsified in the presence of water or an aqueous solution to which an alkali is added. The alkaline reagent includes hydroxides of alkali metals such as NaOH and KOH, compounds of alkaline earth metals, alkaline metal carbonates such as $Na_2CO_3$ and $NaHCO_3$, ammonia, alkaline metal carbonates such as $NH_4CO_3$, and the like.

In the liquid phase oxidation, a mixture thereof to be subjected to oxidation is usually mixed by using a proper agitator for promoting resolution to the liquid component of an oxygen-containing gas or for mixing sufficiently homogeneously the liquid therewith. In addition, without using mechanical means such as an agitator, a method of filling a liquid to be reacted in a column such as a bubble column followed by reacting it by blowing the gas from the bottom of the column, and further a method of inserting downcomers, sieve trays or packings in the column are carried out industrially. Furthermore, a combination of an agitator with a bubble column is also used.

When a reactor equipped with an agitator is used, the agitator is positioned in a reaction mixture and composed of rotating impellers, and the oxidation reaction can be efficiently carried out because of dispersing of an oxygen-containing gas and improving of efficiency of gas-liquid contact, given by effective mixing.

When the dispersity of the gas is insufficient, the efficiency of gas-liquid contact lowers and the reaction efficiency also decreases.

Further, when supply of oxygen becomes insufficient, by-products, polymeric impurities and the like are produced, therefore the yield of the desired product decreases. When the mixing of the liquid is not uniform, the yield of the desired product similarly decreases. Particularly, in emulsion oxidation, when the mixing of the liquids is not uniform, the effect cannot be put out sufficiently. However, when the agitation power is too large, economical and stable operations are interfered in view of the industrial operation because of foaming of the liquid, decrease of a reaction volume caused by increase of foaming, further, increase of more than necessity of electricity consumption, and causing of serious overload to a motor.

Moreover, in general, without using mechanical means such as an agitator in the reaction apparatus, methods of reacting through blowing of the gas into a liquid to be reacted using a bubble column, of inserting downcomers, sieve trays or packings into the column, and of re-circulating in the column a part of the liquid reaction mixture which is drawn outside the column, are carried out industrially. These methods are similar in the purpose to those using an agitator, and are carried out for improving efficiency in contact of a gas with a liquid, uniform mixing of the liquid and reaction efficiency.

The present inventors found as results of intensive studies that a gas component in a gas phase could be effectively dissolved into a liquid phase and the yield of the target component could be maintained at high level by carrying out the mixing under an agitation power of 10.0 kg · m/sec/m$^3$ per unit volume or more, not depending on mixing methods described above.

In the present invention, "agitation power" is indicated by the following equation when the agitation means is mechanical equipment such as an agitator, pump or the like, and a motor is used as power:

$$Z_A = 102 \sum_{m=0}^{m} (W_m/V_m) \qquad (1),$$

In the equation (1), $Z_A$ represents a total agitation power (kg · m/sec/m$^3$), W represents used electric power (kW) of a motor of an apparatus, V represents a reaction volume(m$^3$), and m represents the number of the equipment (motor).

In addition, when any mechanical work such as an agitator is not provided, the agitation power is calculated by using the following equation:

$$Z_B = \{nRT\log_e(P_B/P_T)\}/V \qquad (2)$$

In the equation (2), $Z_B$ represents a total agitation power (kg · m/sec/m$^3$), n is mole number (mol) of a gas supplied into the apparatus, R is 8.314 (J/mol · K) as gas constant, T is temperature (K), $P_B$ is a pressure (Pa) at a liquid surface at which the gas is supplied, V represents a reaction volume (m$^3$). The agitation power per unit volume defined in the present invention is a total value of the values $Z_A$ and $Z_B$ determined in the equations (1) and (2), respectively.

The agitation power per unit volume is preferably 20 to 300 kg · m/sec/m$^3$.

The oxidation method in the present invention can be applied to any one of a batch method and a continuous method.

**EXAMPLE**

Example 1

Oxidation of meta-diisopropylbenzene by air was continuously carried out using a reactor shown in Fig. 1.

The reaction temperature was kept stably at 93°C. At this time, the amount of a hydroperoxide produced per the amount of meta-diisopropylbenzene converted (hereinafter, referred to as "oxidation yield") was 77%. Further, at the time, the total agitation power calculated by the equations (1) and (2) was 51 kg · m/sec/m$^3$.

Comparative Example 1

The reaction was carried out in the same manner as in Example 1 except that the pump was not used. At this time, the oxidation yield was 73%. Further, at the time, the total agitation power calculated by the equations (1) and (2) was 9.5 kg · m/sec/m$^3$.

Comparative Example 2

The reaction is carried out in the same manner as in Example 1 except that an agitator is used a lower part of the column.

When the agitation power calculated by the equation (1) is set to 600 kg · m/sec/m$^3$ , it is speculated that the operation becomes impossible because of foaming of the liquid at the upper liquid level.

**INDUSTRIAL APPLICABILITY**

As described above, according to the present invention, there can be provided a method for oxidizing a liquid component with an oxygen-containing gas, and a method for oxidation of the liquid component, having an excellent characteristic that the oxidation can be carried out economically and stably under high yield.

**Claims**

1.   A process for oxidizing a liquid component with an oxygen-containing gas, wherein the oxidation is carried out under a condition of an agitation power per unit volume of 10.0 to 500 kg·m/sec/m$^3$.

2.   The process according to claim 1, wherein the liquid component is a hydrocarbon, and a product is an organic peroxide of the hydrocarbon.

3.   The process according to claim 1, wherein the liquid component is cumene or meta-diisopropylbenzene.

4.   The process according to claim 1, wherein the oxidation is carried out under a condition in which the liquid component is emulsion.

**Fig.1**

# EP 1 484 298 A1

## INTERNATIONAL SEARCH REPORT

| | International application No. |
|---|---|
| | PCT/JP03/01286 |

### A. CLASSIFICATION OF SUBJECT MATTER
Int.Cl⁷ C07B33/00, C07C407/00, 409/10, 409/12

According to International Patent Classification (IPC) or to both national classification and IPC

### B. FIELDS SEARCHED
Minimum documentation searched (classification system followed by classification symbols)
Int.Cl⁷ C07B33/00, C07C407/00, 409/00

Documentation searched other than minimum documentation to the extent that such documents are included in the fields searched

Electronic data base consulted during the international search (name of data base and, where practicable, search terms used)
CA(STN)

### C. DOCUMENTS CONSIDERED TO BE RELEVANT

| Category* | Citation of document, with indication, where appropriate, of the relevant passages | Relevant to claim No. |
|---|---|---|
| X<br>Y | JP 63-264435 A (Sumitomo Chemical Co., Ltd.),<br>01 November, 1988 (01.11.88),<br>Pages 2 to 5<br>(Family: none) | 1<br>1-4 |
| Y | JP 2001-131124 A (Mitsubishi Chemical Corp.),<br>15 May, 2001 (15.05.01),<br>Pages 3 to 7<br>(Family: none) | 1-4 |
| Y | JP 2001-58974 A (Mitsubishi Chemical Corp.),<br>06 March, 2001 (06.03.01),<br>Pages 2 to 6<br>(Family: none) | 1-4 |

☒ Further documents are listed in the continuation of Box C. ☐ See patent family annex.

| | | |
|---|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family | |

| Date of the actual completion of the international search | Date of mailing of the international search report |
|---|---|
| 04 June, 2003 (04.06.03) | 17 June, 2003 (17.06.03) |

| Name and mailing address of the ISA/<br>Japanese Patent Office | Authorized officer |
|---|---|
| Facsimile No. | Telephone No. |

Form PCT/ISA/210 (second sheet) (July 1998)

6

| INTERNATIONAL SEARCH REPORT | International application No. |
|---|---|
| | PCT/JP03/01286 |

C (Continuation). DOCUMENTS CONSIDERED TO BE RELEVANT

| Category* | Citation of document, with indication, where appropriate, of the relevant passages | Relevant to claim No. |
|---|---|---|
| Y | EP 816335 A1 (General Electric Co.), 07 January, 1998 (07.01.98), & JP 10-87609 A  & US 5767322 A & US 5908962 A | 1-4 |

Form PCT/ISA/210 (continuation of second sheet) (July 1998)

7